# FASCICULE DE BREVET EUROPEEN

(11) **EP 1 099 753 B1**
(45) Date de publication et mention de la délivrance du brevet: **01.03.2006**
(21) Numéro de dépôt: 00403042.5
(22) Date de dépôt: 02.11.2000
(51) Int. Cl.: C12N 1/20, C02F 3/34

(54) **Procédé de production de Burkholderia cepacia dans un milieu contenant du cobalt et son utilisation pour la dégradation de TBA ou de TAA**
Verfahren zur Züchtung von Burkholderia cepacia in einem Kobalt-enthaldenden Medium und seine Verwendung zum Abbau von TBA oder TAA
Method for growing Burkholderia cepacia in a cobalt-containing medium and use thereof for degradating TBA or TAA

(30) Priorité: 09.11.1999 FR 9914212
(43) Date de publication de la demande: 16.05.2001
(73) Titulaire: Institut Français du Pétrole, 92852 Rueil-Malmaison Cedex (FR)
(72) Inventeur: Piveteau, Pascal, 92500 Rueil Malmaison (FR); Fayolle, Françoise, 92140 Clamart (FR); Monot, Frédéric, 92000 Nanterre (FR)

(56) Documents cités:
- EP-A- 0 237 002
- DE-A- 19 962 722
- FR-A- 2 735 497
- FR-A- 2 766 478
- CRIDDLE, C. S. ET AL.: "Transformation of Carbon Tetrachloride by Pseudomonas sp. Strain KC under Denitrification Conditions." APPL. ENVIRON. MICROBIOL., vol. 56, no. 11, novembre 1990 (1990-11), pages 3240-3246, XP000929474

## Description

L'invention concerne un procédé de production de microorganismes, Burkholderia cenacia (ex Pseudomonas cepacia) CIP 1-2052, l'inoculum produit et son application dans un procédé pour dégrader les alcools produits par exemple lors de la dégradation des éthers dits éthers-carburants lorsqu'ils sont contenus dans des effluents aqueux. Ces éthers sont les suivants l'éthyl *tertio*-butyl éther ci-après désigné sous le terme ETBE, le méthyl *tertio-*butyl éther ci-après désigné sous le terme MTBE ou le *tertio*-amyl méthyl éther ci-après désigné sous le terme TAME. Les alcools produits lors de leur dégradation sont le *tertio-*butanol ci-après désigné sous le terme TBA ou l'alcool *tertio*-amylique ci-après désigné sous le terme TAA.

Elle s'applique particulièrement à l'industrie du traitement de l'eau.
L'art antérieur est illustré par le document "Transformation of carbon tetrachloride by Pseudomonas sp; strain KC under denitrification conditions". Appl. Environ Microbiol; vol 56, n°11, Nov. 1990 (1990-11), p. 3240-3246, et par les brevets EP 0 237 002 A, FR 2 735 497 A et FR 2 766 478.

Le TBA et le TAA sont respectivement les produits de dégradation des éthers, MTBE et ETBE d'une part et TAME d'autre part. Ces éthers sont actuellement utilisés ou sont susceptibles d'être utilisés comme additifs dans les essences dont ils permettent d'augmenter l'indice d'octane. Leur utilisation est massive puisque, par exemple, le MTBE est ajouté aux essences à raison de 10 à 15% (v/v). De ce fait, la présence de ces éthers dans les aquifères est de plus en plus souvent notée (Andrews C., 1998, "MTBE-A Long-Term Threat to Ground Water Quality", Ground Water, 36:705-706)). La biodégradation partielle de ces éthers-carburants dans les aquifères, due par exemple à des limitations en oxygène disponible, peut donc aboutir à l'accumulation de TBA et de TAA comme contaminants secondaires.

Par ailleurs, le TBA et le TAA peuvent eux-mêmes être utilisés comme additifs dans les essences pour augmenter leur indice d'octane et dans ce cas leur utilisation peut entrainer leur dispersion dans l'environnement comme contaminants primaires.

La présence de TBA ou de TAA dans les eaux des nappes aquifères servant à l'alimentation en eau potable ou dans les eaux résiduaires arrivant dans les stations d'épuration nécessite la mise en oeuvre de microorganismes spécialisés aptes à dégrader ces alcools qui sont relativement résistants à la biodégradation du fait de leur structure branchée.

Selon le brevet US 4855051, des bactéries isolées du sol, B.coagulans ATCC 53595, A.globiformis ATCC 53596 et P.stutzeri ATCC 53602, sont susceptibles de dégrader le TBA additionné à un milieu minéral minimum. Des consortia bactériens sont également susceptibles de dégrader le TBA selon le brevet US 5811010, mais les microorganismes les constituant n'ont pas été caractérisés. Par ailleurs, selon le brevet US 5814514, différentes bactéries capables de croissance sur propane sont capables de dégrader le TBA.

La Demanderesse a isolé une bactérie aérobie Burkholderia cepacia CIP I-2052 pour sa capacité à utiliser le TBA comme source de carbone et d'énergie en le dégradant jusqu'en dioxyde de carbone (minéralisation) Cette bactérie a été déposée par la Demanderesse à la collection de l'Institut Pasteur (CNCM, 25, rue du Docteur-Roux, F-75724 PARIS). Cette bactérie, selon la demande de brevet FR 2766478 de la Demanderesse, peut être utilisée en culture mixte avec des bactéries capables d'utiliser l'ETBE comme source de carbone et d'énergie en le dégradant jusqu'au stade du TBA qui s'accumule alors dans le milieu de culture; c'est pourquoi l'addition d'un inoculum de Burkholderia cepacia CIP I-2052, ayant la capacité de pousser sur le TBA ainsi produit, permet d'obtenir la dégradation totale de l'ETBE dans des effluents. Généralement, la durée d'incubation de ces microorganismes sur ce substrat est longue, par exemple il faut 400 h pour dégrader environ 700 mg/L de TBA, et il s'avère indispensable à l'échelle industrielle de produire de la biomasse en quantité suffisante.

L'objet de la présente invention est de décrire une modification apportée au milieu de culture de la bactérie Burkholderia cepacia CIP 1-2052 qui permet d'améliorer de manière très simple sa croissance en présence de TBA ou TAA fourni comme seule source de carbone et d'énergie par addition d'un sel de cobalt, seul ou en mélange, dans le milieu de culture.

Plus précisement, l'invention concerne un procédé de production d'une bactérie Burkholderia cepacia CIP I-2052 dans lequel on met en culture, en présence d'air ou d'oxygène, ladite bactérie en présence d'un milieu contenant au moins une source d'azote, du *tertio*-butanol (TBA) et/ou de l'alcool *tertio*-amylique (TAA), et on récupère un inoculum, caractérisé en ce que ledit milieu contient au moins un sel de cobalt. Le cobalt est de préférence utilisé sous sa forme divalente.

Selon une caractéristique du procédé, le sel de cobalt peut être choisi dans le groupe formé par l'ion chlorure, l'ion sulfate, l'ion nitrate et leur mélange Il a été observé que le chlorure de cobalt hexahydraté avait un effet important sur la biomasse produite par les bactéries.

Selon une autre caractéristique de l'invention, la bactérie Burkholderia cepacia CIP 1-2052 peut être ensemencée sur un milieu de culture salin vitaminé auquel on ajoute du chlorure de cobalt, seul ou en mélange avec d'autres oligoéléments, à la concentration finale dans le milieu de 0,01 à 4 mg/L, avantageusement de 0,03 à 2 mg/L et préférentiellement de 0,05 à 0,1 mg/L. Dans ces conditions, les substrats carbonés de Burkholderia cepacia CIP I-2052, c'est-à-dire le TBA ou le TAA peuvent être additionnés à une concentration comprise entre 0,001 et 10 g/L de milieu et de façon préférentielle entre 0,2 et 5 g/L.

Dans ces conditions, on produit un inoculum de densité cellulaire plus importante que ce qui avait été précédemment observé et qui est d'une concentration en biomasse finale, par exemple, de l'ordre de 0,6 g de poids sec/L de culture pour une concentration initiale en TBA de 1 g/L. L'inoculum ainsi préparé pourra être utilisé seul pour traiter des effluents contenant du TBA ou du TAA ou il pourra aussi être utilisé en culture mixte avec des bactéries dégradant l'ETBE ou le MTBE en TBA ou le TAME en TAA.

Cet inoculum ainsi produit en grande quantité par le procédé selon l'invention pourra également être utilisé directement pour épurer des effluents aqueux contenant du TBA ou du TAA ou le mélange de TBA et de TAA. Plus précisement, l'invention concerne un procédé de dégradation de TBA et/ou de TAA, contenu dans des effluents aqueux dans lequel on met en oeuvre dans des conditions aérobies un inoculum produit par le procédé de production d'une bactérie Burkholderia cepacia CIP I-2052.

Selon la demande de brevet FR 98/16520 de la Demanderesse, le TBA peut provenir de la dégradation dans des conditions aérobies, de MTBE ou de l'ETBE contenu dans des effluents aqueux, par au moins une bactérie qui peut être Gordona terrae CIP I-1889 ou Rhodococcus equi CIP I-2053. Ces mêmes bactéries peuvent aussi dégrader dans des conditions aérobies le TAME en TAA.
L'invention sera mieux comprise au vu des figures illustrant l'invention, parmi lesquelles:
- la figure 1 représente la dégradation du TBA par B. cepacia CIP I-2052 et sa croissance P(ufc mL⁻¹) en présence de la solution d'oligo-éléments, en fonction du temps
- la figure 2 montre l'effet de certains éléments minéraux sur la production de biomasse par B. cepacia CIP I-2052.

### EXEMPLES 1A et 1B (comparatif)

On ensemence la souche B. cepacia CIP I-2052 sur milieu minéral salin MM2 supplémenté en glucose (500 mg.L⁻¹) comme source de carbone et d'énergie. Le milieu MM2 a la composition suivante :
- KH₂PO₄ 1,4 g
- K₂HPO₄ 1,7 g
- NaNO₃ 1,5 g
- MgSO₄, 7H₂O 0,5g
- CaCl₂, 2H₂O 0,04 g
- FeCl₃, 6H₂O 0,012 g
- Solution de vitamines 1 mL
- H₂O q.s.p.1 litre

La solution de vitamines a la composition suivante pour 1 litre d'eau distillée .
- Biotine 200 mg
- Riboflavine 50 mg
- Acide nicotinamique 50 mg
- Panthoténate 50 mg
- Acide p-aminobenzoique 50 mg
- Acide folique 20 mg
- Thiamine 15 mg
- Cyanocobalamine 1,5 mg

La culture obtenue après 24 heures d'incubation à 30°C sera utilisée pour ensemencer des fioles contenant 200 mL de milieu MM2 supplémenté en TBA comme source de carbone et d'énergie à différentes concentrations et auxquelles on ajoute une solution d'oligoéléments, contenant du chlorure de cobalt hexahydraté, à raison de 10 mL L⁻¹ de milieu MM2. A titre comparatif, on n'ajoute pas dans ces mêmes fioles de solution d'oligoéléments.

La solution d'oligoéléments a la composition suivante :
- Acide nitrilotriacétique 1,5 g
- Fe(NH₄)₂(SO₄)₂, 6 H₂O 0,2 g
- Na₂SeO₃ 0,2 g
- CoCl₂, 6 H₂O 0,1 g
- MnSO₄, 2H₂O 0,1 g
- Na₂MoO₄, 2H₂O 0,1 g
- ZnSO₄, 7 H₂O 0,1 g
- AlCl₃, 6H₂O 0,04 g
- NiCl₂, 6 H₂O 0,025 g
- H₃BO₃ 0,01 g
- CuSO₄, 5 H₂O 0,01 g
q.s p. 1 litre d'eau distillée.

Chaque fiole est ensemencée à partir de la préculture obtenue à 3% (v/v) et incubée à 30°C sur un incubateur agité.

Sur ces différentes fioles, on effectue des prélèvements à intervalle régulier afin de doser le TBA résiduel par chromatographie en phase gazeuse, on mesure également la densité optique à 600 nm et enfin lorsque tout le TBA initial a été consommé, on effectue une mesure de carbone organique total (COT) montrant le degré de minéralisation du TBA.

Les résultats obtenus avec les différentes cultures sont présentés dans le tableau 1 montrant l'effet de l'addition d'oligoéléments sur la croissance de B cepacia CIP I-2052 en présence de TBA comme source de carbone.

**Tableau 1**

| **Ex** | Supplémentation effectuée | TBA initial (mg.L⁻¹) | TBA final (mg.L⁻¹) | COT final (mg.L⁻¹) | Durée (h) | D.O. initiale à 600 nm | D.O finale à 600 nm |
|---|---|---|---|---|---|---|---|
| **1A** | +oligoéléments | 346,5 | 0 | <10 | 72 | 0,116 | 0,54 |
| | +oligoéléments | 720 | 0 | <10 | 139 | 0,117 | 0,95 |
| | +oligoéléments | 1000,5 | 0 | <10 | 139 | 0,122 | 1,1 |
| **1B** | -oligoéléments | 365,5 | 0 | <10 | 150 | 0,1 | 0,156 |
| | -oligoéléments | 755,1 | 77,5 | N.D.* | 400 | 0,1 | 0,132 |
| | -oligoéléments | 986,8 | 156,2 | N.D.* | 400 | 0,1 | 0,076 |

| | | | | | | | |
|---|---|---|---|---|---|---|---|
| *N.D : non déterminé. | | | | | | | |

Comme on le voit sur ce tableau, l'addition de la solution d'oligoéléments contenant du cobalt permet d'obtenir une dégradation totale du TBA fourni jusqu'à une concentration par exemple de 1000 mg L⁻¹ en diminuant beaucoup la durée d'incubation nécessaire. Par ailleurs, la biomasse produite est beaucoup plus importante comme le montrent les mesures de densité optique. La préparation des inoculums pour des procédés de traitement d'effluents contenant du TBA en quantité importante, par exemple jusqu'à plusieurs g/L, est ainsi plus aisément réalisée.

### EXEMPLE 2

La croissance de la souche B. cepacia CIP I-2052 sur TBA fourni comme seule source de carbone a été suivie en fermenteur de 4L.

On réalise un inoculum de 24 heures de P.cepacia CIP I-2052 sur 200 mL de milieu MM2 contenant du glucose (1 g.L⁻¹) et supplémenté en oligoéléments (1%, v/v) à 30°C sous agitation.

Le milieu de culture du fermenteur était le milieu MM2 décrit dans l'exemple 1 supplémenté avec la solution d'oligoéléments décrite dans l'exemple 1 à raison de 1% (v/v). La concentration initiale en TBA dans le fermenteur était de 300 mg.L⁻¹. Après ensemencement, la D.O. était de 0,05 et la numération, effectuée par numération des étalements sur boites de milieu Luria après dilutions successives, fait apparaitre une densité cellulaire initiale de 2,9 x 10⁶ ufc.mL⁻¹

Les conditions opératoires de cette fermentation en batch étaient les suivantes :
- Température = 30°C
- Aération = 4L.L⁻¹
- pH = 6,8.

La dégradation du TBA est suivie par dosage en CPG sur des surnageants de la culture et on suit également l'évolution de la population bactérienne par numération sur boites de milieu Luria ainsi que par mesure de la densité optique.

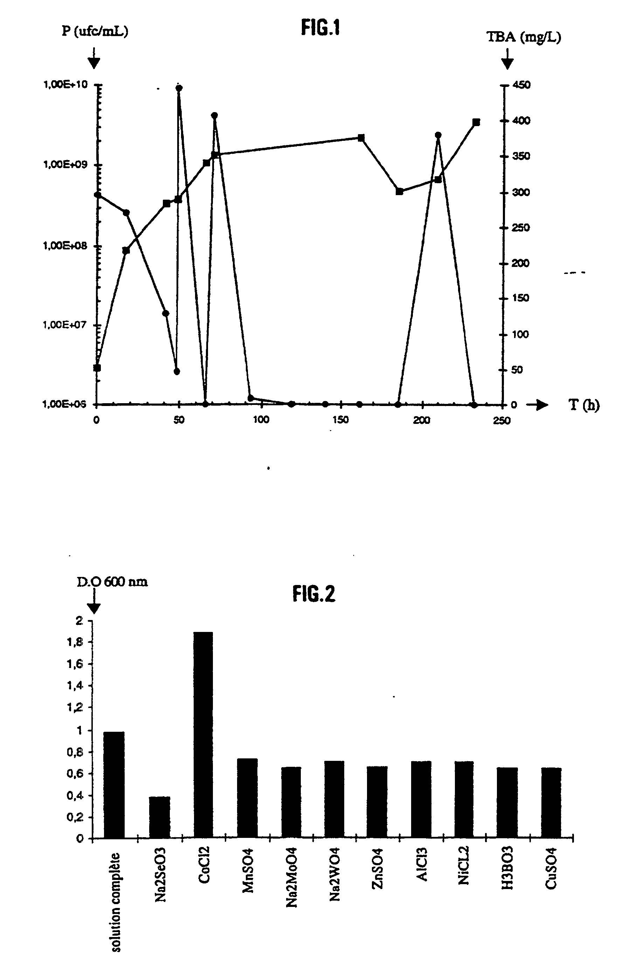

Les résultats sont présentés dans la figure 1 où les additions successives de substrat sont réalisées à 50 heures, 70 heures et 210 heures.

Après 50 heures de culture, tout le TBA initialement fourni était consommé. On réalise alors une première addition de TBA à la concentration de 450 mg.L⁻¹ puis, après consommation, une deuxième addition de 400 mg.L⁻¹ et une troisième addition de 380 mg L⁻¹ sont effectuées.

La population finale obtenue est de 3,5 x 10⁹ ufc mL⁻¹ pour une D.O. à 600 nm finale de 7,0

On voit bien dans cette exemple qu'il est possible d'obtenir facilement des inoculums pouvant être utilisés à des fins de traitement d'effluents contaminés par exemple après fixation des bactéries ainsi obtenues sur un support, par exemple sur un support minéral sur lequel on fait percoler les effluents à traiter, pour une mise en oeuvre de type "biofiltre", décrite dans la demande de brevet FR 2 787 783 de la Demanderesse.

### EXEMPLE 3

On ensemence la souche B. cepacia CIP I-2052 sur milieu minéral salin MM2 décrit dans l'exemple 1 et supplémenté en glucose (500 mg.L⁻¹) comme source de carbone et d'énergie. Cette première culture va être utilisée pour ensemencer 200 mL de milieu MM2 sans addition d'oligoéléments et supplémenté en TBA (750 mg.L⁻¹). Cette préculture, sans addition d'oligoéléments, permet d'épuiser le milieu en éléments minéraux afin de ne pas amener de traces d'oligoéléments dans la culture finale , ce qui fausserait les résultats obtenus.

La préculture ainsi obtenue après 96 heures d'incubation à 30°C sera utilisée pour ensemencer des fioles contenant 200 mL de milieu MM2 supplémenté en TBA comme source de carbone et d'énergie à la concentration de 750 mg.L⁻¹. À chacune des fioles on ajoute un et un seul des éléments entrant dans la composition de la solution d'oligoéléments décrite dans l'exemple 1 à la même concentration finale que lors de l'utilisation de la solution elle-même.

Chaque fiole est ensemencée à partir de la préculture obtenue à 3% (v/v) puis incubée à 30°C sur un incubateur agité.

Sur ces différentes fioles, on mesure la densité optique à 600 nm après 72 heures d'incubation.

Les résultats obtenus avec les différentes cultures sont présentés dans la figure 2.

Ainsi qu'on le voit dans cette figure, l'addition de la solution d'oligoéléments a l'effet positif noté dans les exemples 1 et 2. L'addition de chlorure de cobalt hexahydraté à la concentration finale dans le milieu de 1 mg.L⁻¹ montre un effet important sur la croissance puisque la densité optique obtenue avec l'addition de chlorure de cobalt seul est environ 2 fois supérieure à celle obtenue avec la solution d'oligoéléments, décrite dans l'exemple 1.

### EXEMPLE 4

On réalise la même expérience que dans l'exemple 3, mais au lieu d'utiliser le TBA comme source de carbone et d'énergie pour la souche B. cepacia CIP I-2052, on utilise le TAA dans la même proportion que dans l'exemple 3. Les résultats obtenus sont reportés dans le tableau 2 et sont identiques à ceux déjà décrits dans l'exemple précédent.

**Tableau 2**

| **Ex** | Supplémentation effectuée | TAA initial (mg.L⁻¹) | TAA final (mg.L⁻¹) | Durée (h) | D.O initiale à 600 nm | D.O. finale à 600 nm |
|---|---|---|---|---|---|---|
| **4A** | +oligoéléments | 700 | 0 | 96 | 0,117 | 0,85 |
| **4B** | -oligoéléments | 725 | 90 | 400 | 0,1 | 0,132 |

## Revendications

1. Procédé de production d'une bactérie Burkholderia cepacia CIP I-2052 dans lequel on met en culture ladite bactérie en présence d'un milieu contenant au moins une source d'azote, du *tertio*-butanol (TBA) et/ou de l'alcool *tertio-*amylique (TAA) en présence d'air ou d'oxygène, et on récupère un inoculum, **caractérisé en ce que** ledit milieu contient au moins un sel de cobalt

2. Procédé selon la revendication 1 dans lequel le sel est choisi dans le groupe formé par l'ion chlorure, l'ion sulfate, l'ion nitrate et leur mélange.

3. Procédé selon l'une des revendications 1 et 2, dans lequel le sel de cobalt est un chlorure de cobalt hexahydraté

4. Procédé selon l'une des revendications 1 et 2, dans lequel le sel est introduit à une concentration finale dans le milieu de 0,01 à 4 mg/L et de préférence de 0,05 à 0,1 mg/L.

5. Procédé selon l'une des revendications 1 à 4, dans lequel le *tertio*-butanol ou l'alcool *tertio*-amylique est à une concentration de 0,001 à 10 g/L de milieu.

6. Procédé selon l'une des revendications 1 à 5 dans lequel ladite bactérie est utilisée en culture mixte.

7. Procédé selon l'une des revendications 1 à 6 dans lequel le cobalt est divalent.

8. Inoculum comprenant Burkholderia cepacia CIP I-2052 produit par un procédé selon l'une des revendications 1 à 7.

9. Procédé de dégradation de *tertio*-butanol et/ou d'alcool *tertio*-amylique contenu dans des effluents aqueux dans lequel on met en culture dans des conditions aérobies un inoculum de Burkholderia cepacia CIP I-2052 selon la revendication 8 ou produit par un procédé selon l'une des revendications 1 à 7.

10. Procédé selon la revendication 9 dans lequel le tertio-butanol est le produit de la dégradation du méthyl *tertio*-butyl éther et de l'éthyl *tertio*-butyl éther et l'alcool *tertio*-amylique est le produit de dégradation du *tertio*-amyl méthyl éther, dans des conditions aérobies, par au moins une bactérie choisie dans le groupe formé par Gordona terrae CIP I-1889 et Rhodococcus equi CIP I-2053.

## Claims

1. A process for producing a Burkholderia cepacia CIP 1-2052 bacterium, in which said bacterium is cultured in the presence of a medium containing at least one source of nitrogen, *tert*-butanol (TBA) and/or *tert*-amyl alcohol (TAA) in the presence of air or oxygen, and an inoculum is recovered, **characterized in that** said medium contains at least one cobalt salt.

2. A process according to claim 1, in which the salt is selected from the group formed by the chloride ion, the sulphate ion, the nitrate ion or a mixture thereof.

3. A process according to claim 1 or claim 2, in which the cobalt salt is a hexahydrated cobalt chloride.

4. A process according to claim 1 or claim 2, in which the salt is introduced in a final concentration in the medium of 0.01 to 4 mg/l, preferably 0.05 to 0.1 mg/l.

5. A process according to any one of claims 1 to 4, in which the concentration of *tert*-butanol or *tert*-amyl alcohol is 0.001 to 10 g/l of medium.

6. A process according to any one of claims 1 to 5, in which said bacterium is used in a mixed culture.

7. A process according to any one of claims 1 to 6, in which the cobalt is divalent.

8. An inoculum comprising Burkholderia cepacia CIP I-2052 produced by a process according to any one of claims 1 to 7.

9. A process for degrading *tert*-butanol and/or *tert*-amyl alcohol contained in aqueous effluents, in which an inoculum of Burkholderia cepacia CIP I-2052 according to claim 8 or product of a process according to any one of claims 1 to 7 is cultured under aerobic conditions.

10. A process according to claim 9, in which the *tert*-butanol is the product from degradation of methyl *tert*-butyl ether and ethyl *tert*-butyl ether and the *tert-*amyl alcohol is the product of degradation of *tert*-amyl methyl ether, under aerobic conditions, by at least one bacterium selected from the group formed by Gordona terrae CIP I-1889 and Rhodococcus equi CIP I-2053.

## Patentansprüche

1. Verfahren zur Züchtung einer Bakterie Burkholderia cepacia CIP I-2052, wobei die genannte Bakterie in Gegenwart eines Mediums kultiviert wird, das mindestens eine Quelle von Stickstoff, von tertiärem Butanol (TBA) und/oder von tertiärem Amylalkohol (TAA) in Gegenwart von Luft oder Sauerstoff enthält, und wobei eine Impfkultur gewonnen wird, **dadurch gekennzeichnet, dass** das genannte Medium mindestens ein Kobaltsalz enthält.

2. Verfahren nach Anspruch 1, wobei das Salz aus einer Gruppe ausgewählt wird, bestehend aus einem Chloridion, einem Sulfation, einem Nitration und einer Mischung daraus.

3. Verfahren nach einem der Ansprüche 1 und 2, wobei das Kobaltsalz ein hexahydriertes Kobaltchlorid ist.

4. Verfahren nach einem der Ansprüche 1 und 2, wobei das Salz in einer Endkonzentration von 0,01 bis 4 mg/l, und bevorzugt von 0,05 bis 0,1 mg/l in das Medium eingeführt wird.

5. Verfahren nach einem der Ansprüche 1 bis 4, wobei das tertiäre Butanol oder der tertiäre Amylalkohol eine Konzentration von 0,001 bis 10 g/l im Medium haben.

6. Verfahren nach einem der Ansprüche 1 bis 5, wobei die genannte Bakterie in einer Mischkultur verwendet wird.

7. Verfahren nach einem der Ansprüche 1 bis 6, wobei das Kobalt zweiwertig ist.

8. Impfkultur, umfassend Burkholderia cepacia CIP I-2052, gezüchtet durch ein Verfahren nach einem der Ansprüche 1 bis 7.

9. Verfahren zum Abbau des tertiären Butanols und/oder des tertiären Amylalkohols, die in wässrigen Abwässern enthalten sind, wobei unter aeroben Bedingungen eine Impfkultur von Burkholderia cepacia CIP I-2052 nach Anspruch 8, oder gezüchtet durch ein Verfahren nach einem der Ansprüche 1 bis 7, kultiviert wird.

10. Verfahren nach Anspruch 9, wobei das tertiäre Butanol ein Abbauprodukt des Methyltertiärbutylethers und des Ethyltertiärbutylethers, und der tertiäre Amylalkohol das Abbauprodukt des tertiären Amylmethylethers, unter aeroben Bedingungen ist, durch mindestens eine Bakterie, ausgewählt aus einer Gruppe, bestehend aus Gordona terrae CIP I-1889 und Rhodococcus equi CIP I-2053.
